# EUROPEAN PATENT APPLICATION

(11) **EP 4 752 895 A1**
(43) Date of publication of application: **03.06.2026**
(21) Application number: 25202497.1
(22) Date of filing: 16.09.2025
(51) Int. Cl.: G16H 20/10

(54) **METHOD AND SYSTEM OF MODELLING A THREE-DIMESIONAL WEARABLE DEVICE FOR ASSISTING TARGETED NASAL DRUG DELIVERY**

(30) Priority: 29.11.2024 IN 202421093802
(71) Applicant: Tata Consultancy Services Limited, Mumbai, Maharashtra 400 021 (IN)
(72) Inventor: AHMAD, Dilshad, 411028 Pune (IN); SINGH, Umesh, 411028 Pune (IN); BASAVARSU, Purushottham Gautham, 411028 Pune (IN)
(74) Representative: Goddar, Heinz J.

(57) **Abstract**

This disclosure relates generally to a method and system for modeling a three-dimensional (3D) wearable device assisting nasal administration of drugs. State-of-the-art methods include leaflets or manuals with a set of instructions for administering the drug. However, this may result in improper administration due to variations in the nasal geometry and manual operations. The disclosed method involves the 3D wearable device that aids in achieving maximum concentration of the drug at the target site. The modeling facilitate an introduction of a collared geometry that provides customed passage to the drug within the nasal cavity based on a plurality of nasal spray specifications. Further, the 3D wearable device model is transformed to a scale based on the CT scan data of the nose to obtain a 3D printable wearable device for the nose.

## Description

### CROSS-REFERENCE TO RELATED APPLICATIONS AND PRIORITY

The present application claims priority from Indian patent application no. 202421093802, filed on Nov 29, 2024.

### TECHNICAL FIELD

The disclosure herein generally relates to targeted nasal drug delivery, and, more particularly, to model a personalized three-dimensional wearable device for the assisted targeted nasal drug delivery.

### BACKGROUND

The nasal route of administration is widely recognized for its effectiveness in drug delivery. It is commonly used for the topical administration of medications, particularly in treating nasal conditions such as nasal congestion. Medications frequently administered this way include decongestants, antihistamines, cromoglycates, steroids, and antibiotics. Additionally, nasal drug delivery has been employed to treat or alleviate inflammation in areas such as the nasopharynx, paranasal sinuses, and auditory tubes.

Of particular note is the potential of nasal delivery for targeting the brain, offering significant prospects for the prevention and treatment of central nervous system (CNS) diseases. By delivering drugs through the nasal cavity, it is possible for them to bypass the blood-brain barrier (BBB) and directly reach the brain, enhancing therapeutic effectiveness while minimizing adverse side effects.

Furthermore, it is used for systemic drug delivery in the treatment of conditions such as migraines, osteoporosis, nausea, and hormone deficiencies. Researchers are also investigating the use of nasal drug delivery for COVID-19 vaccinations. The primary method for local, systemic, and CNS drug delivery via the nasal route is through nasal sprays, making it a promising and efficient approach to drug therapy.

Despite numerous advantages associated with the delivery of medicaments via nasal route, drug containers as well as nasal drug delivery devices come with certain limitations. E.g. traditional nasal droppers require difficult maneuvers by the patient for correct administration and optimal deposition. The spray bottles comprising drugs meant for nasal administration comes with several instructions to administer a dose, such as a prescribed orientation and position of the nose tip.

According to the marketed nasal products, nasal drug preparations include nasal drops, nasal sprays, nasal aerosols and nasal ointments/creams. Marketed nasal ointments and creams are limited and employed mainly for the therapy of nasal bacterial infection and relief of nasal congestion due to their longer retention time. One of the challenges with nasal drug delivery is to achieve a uniform dose of drug as well as to direct the delivery to the target site so that faster and effective results can be obtained. Nasal aerosols and nasal sprays are effective in targeted drug delivery as they facilitate drug outreach to its target site. However, the nasal aerosols and nasal sprays suffer from non-uniform or under delivery of drug due to dissemination of the drug as mist in the surroundings. Similarly, drug actuated/ sprayed from the nasal aerosols and sprays may not hit the target site. Therefore, it is essential to direct the drug delivery to the target site or to the closest proximity to the target site.

### SUMMARY

Embodiments of the present disclosure present technological improvements as solutions to one or more of the above-mentioned technical problems recognized by the inventors in conventional systems. For example, in one embodiment, a method of modelling a 3D wearable device for assisting nasal drug delivery is provided. The method includes receiving a three dimensional (3D) wearable device model of a desired thickness, wherein one or more quality specifications are utilized to estimate the desired thickness of the 3D wearable device model. To create the 3D wearable device model of the desired thickness (a) one or more two dimensional (2D) facial images of a subject, and (b) a CT scan data of nose of the subject are received by the 3D wearable device module. Then, a plurality of feature points corresponding to the nose of the subject are extracted from the one or more 2D facial images of the subject. Then a zero-thickness nose boundary surface file is generated using the extracted plurality of feature points. Further, a desired thickness to the zero-thickness nose boundary surface file is imparted to obtain the three dimensional (3D) wearable device model, wherein one or more quality specifications are utilized to estimate the desired thickness of the 3D wearable device model. Further, the method includes receiving a plurality of specifications corresponding to a prescribed nasal spray, wherein the plurality of specifications include (a) a nasal spray geometry, (b) a nasal spray tip position, and (c) a nasal spray orientation. Further, the method includes integrating a collar geometry to the 3D wearable device model by incorporating a cylinder on each nostril region wherein a diameter of the cylinder is based on one or more quality parameters, and wherein each cylinder undergoes a plurality of operations to (a) align based on one or more orientation vectors of the 3D wearable device model, (b) slice based on a pre-defined collar geometry height, (c) unite with the 3D wearable device model, and (d) remove a region from the 3D wearable device model that superimposes the nasal spray tip dimensions. The scaling of the 3D wearable device model based on the plurality of specifications corresponding to the prescribed nasal spray generates one or more collars within the 3D wearable device, wherein the one or more collars assists in a nasal drug ingestion to a target site within the nose. The collar generation includes creating one or more cylinders along one or more nostril openings using the nasal spray tip position and the nasal spray orientation corresponding to the prescribed nasal spray. Further, one or more cylinders are aligned to the nasal spray orientation wherein one or more cylinders are transformed based on a transformation matrix. The transformed cylinders are sliced by inflated 3D wearable device model along a plurality of surface points to generate plurality of intersection volumes of collar geometry. The collar geometry is integrated to the 3D wearable device model. From the 3D wearable device model having the integrated collar geometry, a region is removed that superimposes the nasal spray tip dimensions. This creates an opening in the 3D wearable device model that facilitates passage of drug through the nasal route to the target site. Further, the method includes transforming the 3D wearable device model to a scale based on the CT scan data of the nose to obtain a 3D printable wearable device for the nose. A 3D face STL file is generated from the CT scan data by classifying the CT scan data based on the pre-defined threshold, and by cleaning with desired features wherein the classified data is cleaned to retain the desired features. The 3D face STL file is generated through segmentation wherein the CT scan data is classified based on the pre-defined threshold corresponding to air to obtain one or more segments relevant for obtaining the 3D face STL file. The transformation matrix is calculated to scale the 3D wearable device model. For the calculation of the transformation matrix, key landmarks from both methods (CT scan and facial image) are utilized. The transformation scales and aligns the 3D holder to fit the real nose size.

In another aspect, a system of modelling 3D wearable device for assisting nasal drug delivery is provided. The system includes at least one memory storing programmed instructions; one or more Input /Output (I/O) interfaces; and one or more hardware processors, and a 3D wearable device module operatively coupled to a corresponding at least one memory, wherein the system is configured receive a three dimensional (3D) wearable device model of a desired thickness, wherein one or more quality specifications are utilized to estimate the desired thickness of the 3D wearable device model. To create the 3D wearable device model of the desired thickness (a) one or more two dimensional (2D) facial images of a subject, and (b) a CT scan data of nose of the subject are received by the 3D wearable device module. Then, a plurality of feature points corresponding to the nose of the subject are extracted from the one or more 2D facial images of the subject. Then a zero-thickness nose boundary surface file is generated using the extracted plurality of feature points. Further, a desired thickness to the zero-thickness nose boundary surface file is imparted to obtain the three dimensional (3D) wearable device model, wherein one or more quality specifications are utilized to estimate the desired thickness of the 3D wearable device model. The system is configured to receive a plurality of specifications corresponding to a prescribed nasal spray, wherein the plurality of specifications include (a) a nasal spray geometry, (b) a nasal spray tip position, and (c) a nasal spray orientation. The system is configured to integrate a collar geometry to the 3D wearable device model by incorporating a cylinder on each nostril region wherein a diameter of the cylinder is based on one or more quality parameters, and wherein each cylinder undergoes a plurality of operations to (a) align based on one or more orientation vectors of the 3D wearable device model, (b) slice based on a pre-defined collar geometry height, (c) unite with the 3D wearable device model, and (d) remove a region from the 3D wearable device model that superimposes the nasal spray tip dimensions. The scaling of the 3D wearable device model based on the plurality of specifications corresponding to the prescribed nasal spray integrates collar geometry to the 3D wearable device model by incorporating a cylinder on each nostril region wherein a diameter of the cylinder is based on one or more quality parameters, and wherein each cylinder undergoes a plurality of operations to (a) align based on one or more orientation vectors of the 3D wearable device model, (b) slice based on a pre-defined collar geometry height, (c) unite with the 3D wearable device model, and (d) remove a region from the 3D wearable device model that superimposes the nasal spray tip dimensions. The scaling of the 3D wearable device model based on the plurality of specifications corresponding to the prescribed nasal spray generates one or more collars within the 3D wearable device, wherein the one or more collars assists in a nasal drug ingestion to a target site within the nose. The collar generation includes creating one or more cylinders along one or more nostril openings using the nasal spray tip position and the nasal spray orientation corresponding to the prescribed nasal spray. Further, one or more cylinders are aligned to the nasal spray orientation wherein one or more cylinders are transformed based on a transformation matrix. The transformed cylinders are sliced by inflated 3D wearable device model along a plurality of surface points to generate an intersection volume of collar geometry. The collar geometry is integrated to the 3D wearable device model. From the 3D wearable device model having the integrated collar geometry, a region is removed that superimposes the nasal spray tip dimensions. This creates an opening in the 3D wearable device model that facilitates passage of drug through the nasal route to the target site. Further, the system is configured to transform the 3D wearable device model to a scale based on the CT scan data of the nose to obtain a 3D printable wearable device for the nose. A 3D face STL file is generated from the CT scan data by classifying the CT scan data based on the pre-defined threshold, and by cleaning with desired features wherein the classified data is cleaned to retain the desired features. The 3D face STL file is generated through segmentation wherein the CT scan data is classified based on the pre-defined threshold corresponding to air to obtain one or more segments relevant for obtaining the 3D face STL file. The transformation matrix is calculated to scale the 3D wearable device model. For the calculation of the transformation matrix, key landmarks from both methods (CT scan and facial image) are utilized. The transformation scales and aligns the 3D holder to fit the real nose size.

In yet another aspect, a computer program product including a non-transitory computer-readable medium embodied therein a computer program of modelling 3D wearable device for assisting nasal drug delivery is provided. The computer readable program, when executed on a computing device, causes the computing device to receive a three dimensional (3D) wearable device model of a desired thickness, wherein one or more quality specifications are utilized to estimate the desired thickness of the 3D wearable device model. To create the 3D wearable device model of the desired thickness (a) one or more two dimensional (2D) facial images of a subject, and (b) a CT scan data of nose of the subject are received by the 3D wearable device module. Then, a plurality of feature points corresponding to the nose of the subject are extracted from the one or more 2D facial images of the subject. Then a zero-thickness nose boundary surface file is generated using the extracted plurality of feature points. Further, a desired thickness to the zero-thickness nose boundary surface file is imparted to obtain the three dimensional (3D) wearable device model, wherein one or more quality specifications are utilized to estimate the desired thickness of the 3D wearable device model. The computer readable program, when executed on a computing device, causes the computing device to receive a plurality of specifications corresponding to a prescribed nasal spray, wherein the plurality of specifications include (a) a nasal spray geometry, (b) a nasal spray tip position, and (c) a nasal spray orientation. The computer readable program, when executed on a computing device, causes the computing device to integrate a collar geometry to the 3D wearable device model by incorporating a cylinder on each nostril region wherein a diameter of the cylinder is based on one or more quality parameters, and wherein each cylinder undergoes a plurality of operations to (a) align based on one or more orientation vectors of the 3D wearable device model, (b) slice based on a pre-defined collar geometry height, (c) unite with the 3D wearable device model, and (d) remove a region from the 3D wearable device model that superimposes the nasal spray tip dimensions. The scaling of the 3D wearable device model based on the plurality of specifications corresponding to the prescribed nasal spray integrates collar geometry to the 3D wearable device model by incorporating a cylinder on each nostril region wherein a diameter of the cylinder is based on one or more quality parameters, and wherein each cylinder undergoes a plurality of operations to (a) align based on one or more orientation vectors of the 3D wearable device model, (b) slice based on a pre-defined collar geometry height, (c) unite with the 3D wearable device model, and (d) remove a region from the 3D wearable device model that superimposes the nasal spray tip dimensions. The scaling of the 3D wearable device model based on the plurality of specifications corresponding to the prescribed nasal spray generates one or more collars within the 3D wearable device, wherein the one or more collars assists in a nasal drug ingestion to a target site within the nose. The collar generation includes creating one or more cylinders along one or more nostril openings using the nasal spray tip position and the nasal spray orientation corresponding to the prescribed nasal spray. Further, one or more cylinders are aligned to the nasal spray orientation wherein one or more cylinders are transformed based on a transformation matrix. The transformed cylinders are sliced by inflated 3D wearable device model along a plurality of surface points to generate an intersection volume of the collar geometry. The collar geometry is integrated to the 3D wearable device model. From the 3D wearable device model having the integrated collar geometry, a region is removed that superimposes the nasal spray tip dimensions. This creates an opening in the 3D wearable device model that facilitates passage of drug through the nasal route to the target site. The computer readable program, when executed on a computing device, causes the computing device to transform the 3D wearable device model to a scale based on the CT scan data of the nose to obtain a 3D printable wearable device for the nose. A 3D face STL file is generated from the CT scan data by classifying the CT scan data based on the pre-defined threshold, and by cleaning with desired features wherein the classified data is cleaned to retain the desired features. The 3D face STL file is generated through segmentation wherein the CT scan data is classified based on the pre-defined threshold corresponding to air to obtain one or more segments relevant for obtaining the 3D face STL file. The transformation matrix is calculated to scale the 3D wearable device model. For the calculation of the transformation matrix, key landmarks from both methods (CT scan and facial image) are utilized. The transformation scales and aligns the 3D holder to fit the real nose size.

In another aspect, a 3D wearable device for the nose of a subject is provided. The 3D wearable device for nose comprises a body having an inner surface designed in synchronization with one or more external aspects of the nose of the subject, and an outer surface having a plurality of collars to direct a nasal drug delivery to a target site within the nose; and a right-side nostril opening and a left-side nostril opening provisioning the nasal spray entry to the nose, and wherein the 3D wearable device is obtained using one or more 3D printing techniques.

It is to be understood that both the foregoing general description and the following detailed description are exemplary and explanatory only and are not restrictive of the invention, as claimed.

### BRIEF DESCRIPTION OF DRAWINGS

The accompanying drawings, which are incorporated in and constitute a part of this disclosure, illustrate exemplary embodiments and, together with the description, serve to explain the disclosed principles:
FIG. 1 illustrates an exemplary block diagram of system 100 for modelling a three-dimensional (3D) wearable device, according to some embodiments of the present disclosure.
FIG. 2 is a flow diagram illustrating preparation of a 3D nasal holder surface for a 3D wearable device model, according to some embodiments of the present disclosure.
FIG. 3 depict schematics of a nose surface file generation from a two-dimensional (2D) facial image of the subject, according to some embodiments of the present disclosure.
FIG. 4 depicts various stages of obtaining the thickened 3D wearable device model from the 3D nasal holder surface of zero thickness, according to some embodiments of the present disclosure.
FIG. 5 is a flow diagram illustrating modeling of the 3D device using nasal spray specifications, according to some embodiments of the present disclosure.
FIG. 6 illustrates a collar introduction mechanism onto a thickened 3D wearable device model, according to some embodiments of the present disclosure.
FIG. 7 illustrates nasal spray provisioning and breathing assistance within the 3D wearable device model, according to some embodiments of the present disclosure.
FIG. 8 is a flow diagram illustrating transforming the 3D wearable device model to a scale corresponding to a CT scan data of the subject, according to some embodiments of the present disclosure.
FIGS. 9A-9B is a flow diagram of an illustrative method 900 modeling the 3D wearable device using the system of FIG. 1, according to some embodiments of the present disclosure.
FIG. 10 depicts the 3D wearable device model generated, according to some embodiments of the present disclosure.

### DETAILED DESCRIPTION

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

The present invention discloses modeling of a three-dimensional (3D) wearable device for assisting nasal administration of drugs. The 3D wearable device aids in achieving maximum concentration of the drug at the target site. The modeling facilitates an introduction of a collared geometry that provides customed passage to the drug within the nasal cavity based on a plurality of nasal spray specifications, facial details and anatomy of the nasal cavity. Currently doctors and nasal spray manufacturers, especially sprays and aerosols manufacturers provide a general instruction to use the product. However, achieving effective drug concentration at the target site delivery by following mere instructions is difficult to achieve due to unguided manual administration, variation in nasal anatomy, and handling of nasal sprays by users. Therefore, a personalized or a customizable aid for an intra-nasal drug delivery is needed. Several advantages associated by directing the drug to the target site within the nasal cavity includes (a) achieving targeted drug delivery through a passage customized based on nasal anatomy as well as nasal spray specifications, (b) consistency of dose delivery over a repeated administration as well as over period of time, (c) quite safe and friendly for kids and elderly, and (d) a personalized solution to achieve an assisted drug delivery at a specified clinical region in the nasal cavity for the subjects having deviation in nasal anatomy. State-of-art nasal spray applicators (such as pump, metered-dose inhaler) lacks accurate drug delivery and user-friendliness as they are based on clinical experiments conducted on three-dimensional (3D) printed nasal cavity. These sprays are generic and do not consider nasal deviation each subject has while devising the nasal applicators. If not used appropriately these nasal applicators result in an irritation on mucosal linings, side-effects and patient discomfort resulting into rejection at the clinical trial phase interventions. In the present disclosure, a 3D wearable device model is generated by considering nasal anatomy of the subject as well as the nasal spray specifications. The method of generating the 3D wearable device model considers a plurality of features extracted from the facial images of the subject, CT scan data of the subject as well as the specifications of the nasal spray prescribed to the subject. A customized solution in terms of the 3D wearable device model assists the subject in achieving maximum drug concentration at the target site within the nasal cavity.

As used herein the term "nasal spray specifications" refers to one or more characteristics associated with a spray container such as (a) a nasal spray geometry, (b) a nasal spray tip position, and (c) a nasal spray orientation.

As used herein the term 'nasal spray' refers to a medication in the form of a liquid or solid particles in a suspended or a dispersed state that are inhaled as a fine spray or mist, and wherein inhalation may be facilitated through metered pump or an actuation mechanism.

As used herein the term 'nasal spray tip position' and 'nasal spray orientation' are the position of the nasal spray tip and orientation of the nasal spray tip respectively so that when spray tip is kept at 'tip position' and axis of the nasal spray is oriented along the orientation the ingested medicine received at the target location is optimum.

As used herein the term '3D wearable device' refers to a personalized device made from 3D printing technique by utilizing a 3D wearable device model generated according to the process of the present disclosure.

As used herein the term '3D wearable device model' refers to a model assisting nasal drug delivery wherein the model is in a form of a STL file and is utilized for generating a 3D wearable device using one or more 3D printing techniques.

As used herein 'quality specifications' refers to one more characteristic associated with the 3D wearable device model such as (a) stiffness of the 3D printed device, (b) material used in the 3D printing of the device, (c) weight of the final device, and (d) surface finish of the device and (e) cost of the final 3D printed device.

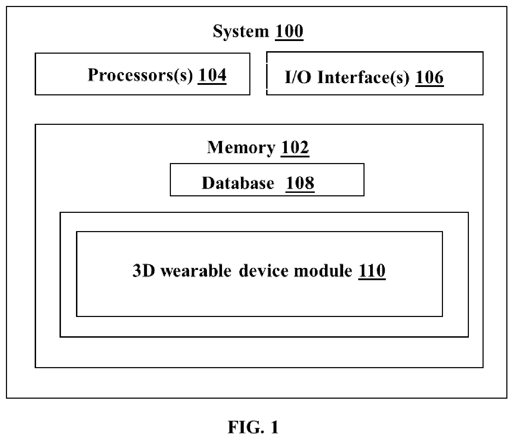

FIG. 1 illustrates an exemplary block diagram of system 100 for modelling a three-dimensional (3D) wearable device, according to some embodiments of the present disclosure.

In an embodiment, the system 100 includes a processor(s) 104, communication interface device(s) 106, alternatively referred as input/output (I/O) interface(s) 106, and one or more data storage devices or a memory 102 operatively coupled to the processor(s) 104. System 100 with one or more hardware processors is configured to execute functions of one or more functional blocks of system 100. Referring to the components of system 100, in an embodiment, the processor(s) 104, can be one or more hardware processors 104. In an embodiment, the one or more hardware processors 104 can be implemented as one or more microprocessors, microcomputers, microcontrollers, digital signal processors, central processing units, state machines, logic circuitries, and/or any devices that manipulate signals based on operational instructions. Among other capabilities, the one or more hardware processors 104 are configured to fetch and execute computer-readable instructions stored in memory 102. In an embodiment, system 100 can be implemented in a variety of computing systems including laptop computers, notebooks, hand-held devices such as mobile phones, workstations, mainframe computers, servers, and the like. The I/O interface(s) 106 can include a variety of software and hardware interfaces, for example, a web interface, a graphical user interface to display the generated target images and the like and can facilitate multiple communications within a wide variety of networks N/W and protocol types, including wired networks, for example, LAN, cable, etc., and wireless networks, such as WLAN, cellular and the like. In an embodiment, the I/O interface (s) 106 can include one or more ports for connecting to a number of external devices or to another server or devices. The memory 102 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes. In an embodiment, memory 102 includes a 3D wearable device module 110. The 3D wearable device module 110 extracts a plurality of feature points corresponding to the nose of the subject from one or more 2D facial images of the subject to generate a zero-thickness nose boundary surface file. The 3D wearable device module 110 generates a three dimensional (3D) wearable device model of a desired thickness using the zero-thickness nose boundary surface file. Further, the 3D wearable device module 110 receives a plurality of specifications corresponding to a prescribed nasal spray wherein the plurality of specifications includes (a) a nasal spray geometry, (b) a nasal spray tip position, and (c) a nasal spray orientation. Further, the modules execute integration of a collar geometry to the 3D wearable device model by incorporating a cylinder on each nostril region wherein a diameter of the cylinder is based on one or more quality parameters, and wherein each cylinder undergoes a plurality of operations to (a) align based on one or more orientation vectors of the 3D wearable device model, (b) slice based on a pre-defined collar geometry height, (c) unite with the 3D wearable device model, and (d) remove a region from the 3D wearable device model that superimposes the nasal spray tip dimensions; and transforming (908), via the one or more hardware processors, the 3D wearable device model to a scale based on the CT scan data of the nose to obtain a 3D printable wearable device for the nose. The collar facilitates directed passage of the drug to the intra-nasal cavity based on nasal spray specifications. The 3D wearable device module 110 transforms the 3D wearable device model to a scale based on the CT scan data of the nose to obtain a 3D printable wearable device for the nose. The memory 102 further comprises of a plurality of modules that includes programs or coded instructions that supplement applications or functions performed by the system 100 for executing different steps involved in the analysis of the health of the subject, being performed by the system 100. The modules, amongst other things, can include routines, programs, objects, components, and data structures, which perform particular tasks or implement particular abstract data types. The modules may also be used as signal processor(s), node machine(s), logic circuitries, and/or any other device or component that manipulates signals based on operational instructions. Further, the modules can be used by hardware, by computer-readable instructions executed by one or more hardware processors 104, or by a combination thereof. The modules may include computer-readable instructions that supplement applications or functions performed by the system 100. Further, the memory 102 may comprise information pertaining to input(s)/output(s) of each step performed by the processor(s) 104 of the system100 and methods of the present disclosure. Further, the memory 102 includes a database 108. The database (or repository) 108 may include a plurality of abstracted piece of code for refinement and data that is processed, received, or generated as a result of the execution of the plurality of modules. The external database is communicatively coupled to the system 100. The data contained within such an external database may be periodically updated. For example, new data may be added into the database (not shown in FIG. 1) and/or existing data may be modified and/or non-useful data may be deleted from the database. In one example, the data may be stored in an external system, such as a Lightweight Directory Access Protocol (LDAP) directory and a Relational Database Management System (RDBMS).

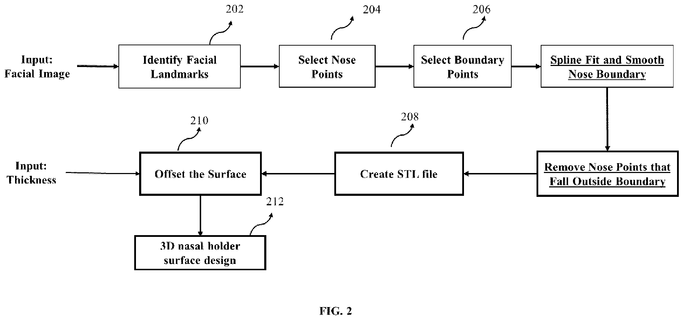

FIG. 2 is a flow diagram illustrating preparation of a 3D nasal holder surface for a 3D wearable device model, according to some embodiments of the present disclosure.

As illustrated in FIG. 2, the 3D nasal holder surface is first designed using computer aided design (CAD) features. The 3D nasal holder surface serves as a base onto which the 3D wearable device model build. At 202, a plurality of facial landmarks are extracted from the facial image input to the system 100. The facial landmarks are obtained using number of techniques such as Active Appearance Models (AAM), Constrained Local Models (CLM), Facial Landmark Detection using Dlib, Deep Learning-Based Approaches (MTCNN, FaceNet), Supervised Descent Method (SDM), Facial Landmark Detection in OpenCV, 3D Morphable Models (3DMM), Feature-based Methods (HOG, SIFT, SURF), MediaPipe Face Mesh and OpenPose.

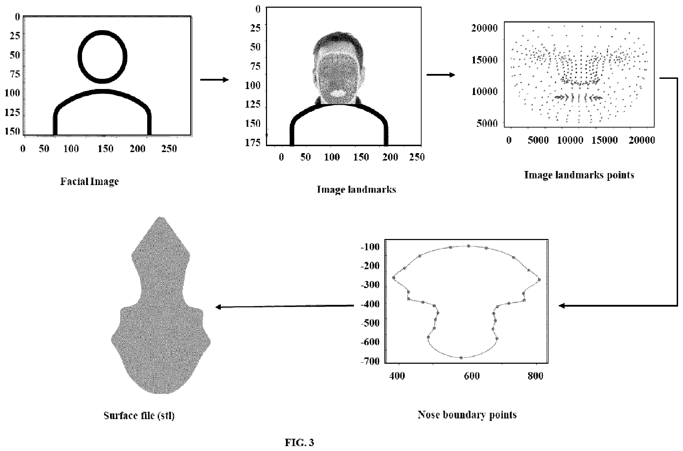

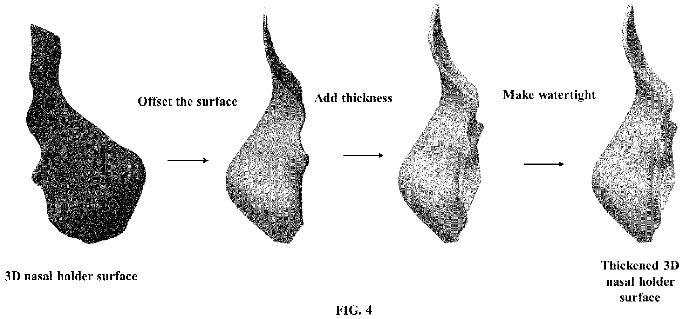

At 204, from the plurality of facial landmarks, a plurality of nose landmarks points are identified. A smooth surface is obtained using one of the suitable smooth functions such as Spline or Polynomial. The identified nose landmark points create a smooth surface around the nose of the subject. At 206 one or more boundary points are identified to obtain a facial boundary points. Further the identified boundary points are taken through one or more processing operations includes applying "Spline Fit and Smooth Boundary" function, and "Remove Points that Fall Outside Boundary" functions to obtain a plurality of nose boundary points. At 208, the plurality of nose boundary points are processed to generate a STL file. At 210, the STL file is subject to offset operation followed by adding a desired thickness and providing a watertight geometry to obtain a thickened 3D nasal holder surface design 212. Schematics of the nose surface file generation from the 2D facial image of the subject is shown in FIG. 3 and various stages of obtaining the thickened 3D nasal holder surface design from the 3D nasal holder surface of zero thickness is presented in FIG. 4.

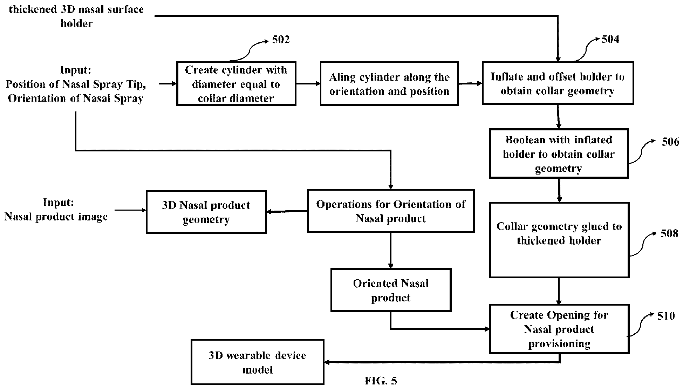

FIG. 5 is a flow diagram illustrating modeling of the 3D device using nasal spray specifications, according to some embodiments of the present disclosure.

As illustrated in FIG. 5, the 3D wearable device model is customized based on the plurality of nasal spray specifications. The thickened 3D nasal surface holder serves as a base to perform customization operations. The plurality of specifications corresponding to a prescribed nasal spray includes (a) a nasal spray geometry, (b) a nasal spray tip dimension, and (c) a nasal spray orientation. The nasal spray specifications are utilized in generating a collar geometry and further integrating the collar geometry to the 3D wearable device model.

At 502, the module 110 receives the nasal spray tip dimension and orientation of the nasal spray in terms of position vector and orientation vectors. Based on the tip dimension, one or more cylinders are created with diameter equal to the collar diameter estimate using quality parameters. These cylinders are then transformed to take position and orientation. A diameter of the cylinder is based on one or more quality parameters, and wherein each cylinder undergoes a plurality of boolean operations to (a) align based on one or more orientation vectors of the 3D wearable device model, (b) slice based on a pre-defined collar geometry height, (c) unite with the 3D wearable device model, and (d) remove a region from the 3D wearable device model that superimposes the nasal spray tip dimensions.

The alignment of the cylinder towards outer surface of the 3D wearable device model is based on alignment vectors including an orientation vector and an axis vector. A transformation matrix is calculated using orientation vector and axis vector of the cylinder. The cylinder thus created is transformed to achieve the required orientation. The slicing involves performing one or more boolean operations on the 3D wearable device model to generate the one or more collars. Two collars of the pre-defined height covering the nostril region are protruded towards outer surface of the 3D wearable device model. The 3D thickened holder surface obtained in the previous step is further inflated and offset using one or more quality parameters. The inflation and offset will result in the height of the collar geometry. The one or more collars assists in a nasal drug ingestion to a target site within the nose. Slicing is a customized approach of making geometric changes to the collar based on nasal spray specifications. The slicing generates an intersection volume of the collared geometry. The system 100 utilizes the nasal spray tip dimensions tip dimension received as one of the nasal spray specifications. After slicing operation, the collar geometry is integrated to the 3D wearable device model by a boolean union operation. The orientation of the nasal spray is achieved by performing transformation using transformation matrix. The transformation matrix is calculated using the nasal spray tip position and orientation and tip position and orientation from the received specifications. Based on the nasal spray tip dimensions , a portion of the 3D wearable model is removed by using the boolean operator that creates an opening for the nasal spray. The boolean subtraction operation is carried out on the 3D wearable device model for the opening along the aligned collar. The opening is customized as per nasal spray tip dimensions and facilitate an assisted delivery of the drug to the target site.

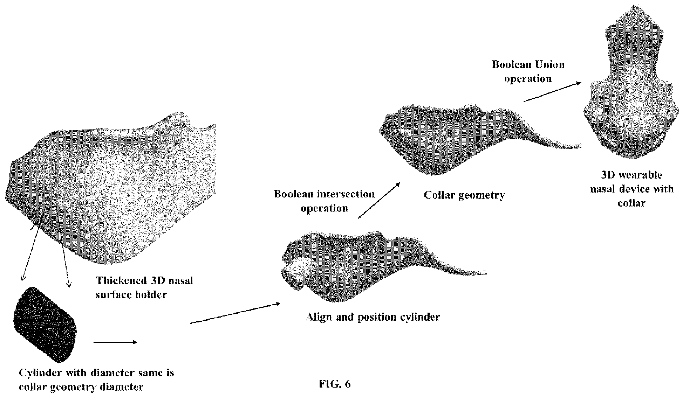

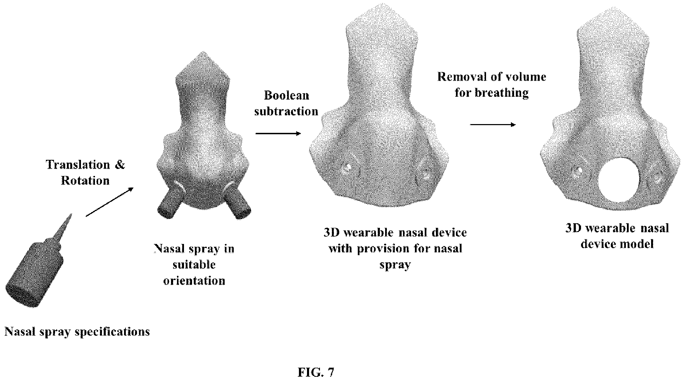

While performing the one or more transformation operations, using a transformation matrix. A transformation matrix is calculated using the orientation vector and cylinder axis. The cylinder thus created is transformed to achieve the required orientation. At 504, 3D thickened holder surface is obtained. This surface is further inflated and offset using one or more quality parameters. The inflation and offset will result in the height of the collar geometry. At 506, A boolean operation is performed between the cylinder and the inflated '3D wearable device model' to slice the cylinder. The slicing generates a collar geometry. At 508, a multiple boolean operations are performed to integrate the collar geometry into a 3D wearable device model to obtain a 3D wearable device model with the collar geometry. At 510, an opening is introduced for the nasal spray provisioning. An opening is created in the collar for nasal spray provisioning using the 'Create Opening for Nasal spray Provisioning' step. This utilizes two inputs: the 3D wearable device model with the collar and an oriented nasal spray. A boolean subtraction operation is carried out on the 3D wearable device model with the collar design where the oriented nasal spray aligns. To obtain the oriented nasal spray design, a nasal spray geometry is first created using the nasal spray image as an input. The orientation of the nasal spray is achieved by performing operations for orientation, that involves calculating the transformation matrix using the nasal spray tip position and orientation and transforming the nasal spray geometry accordingly. This feature embed a full functionality to the 3D wearable device model. Some volume is further removed to provide space for breathing air. The collar introduction mechanism onto the thickened 3D wearable device model is presented in the FIG. 6 and nasal spray provisioning and breathing assistance within the 3D wearable device model is presented in the FIG. 7.

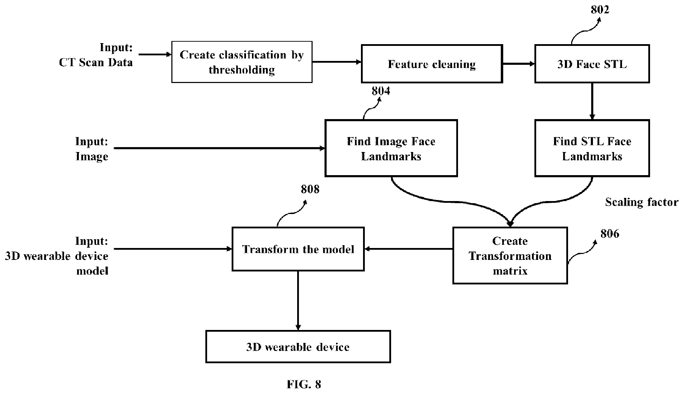

FIG. 8 is a flow diagram illustrating transforming the 3D wearable device model to a scale corresponding to a CT scan data of a subject, according to some embodiments of the present disclosure.

At 802, a 3D face STL file is generated from the CT scan data received by the system 100. STL is a file format commonly used for 3D printing and computer-aided design (CAD). The CT scan data of the subject's head is processed to create a 3D face of the subject. The generation of the 3D face STL file requires two steps: (i) classification of 3D scan data wherein a pre-defined thresholding is applied to classify the scan data, and (ii) cleaning with desired features wherein the classified data is cleaned to retain the desired features.

The 3D face of the subject using CT data is generated by using threshold value corresponding to the air threshold value in the CT data. This threshold value provides 3D profile of the face corresponding to skin of the subject. The thresholding also results into various volumes inside head. These volumes are dropped based on the fact that volume size corresponding to the face has largest size as compared to other volumes in the head generated using thresholding. The resulting geometry is saved in STL format and processed to generate a first facial landmark comprising a plurality of 3D facial datapoints of the entire face.

At 804, the 2D facial image is separately processed to generate a second facial landmark comprising a plurality of 2D facial datapoints of the entire face. The landmark identification is done using techniques on STL file or alternatively a STL is given a skin color and facial snap is generated. At 806, the first facial landmark and the second facial landmark are combined to generate a transformation matrix. The transformation matrix is calculated to scale the 3D wearable device model. For the calculation of the transformation matrix, key landmarks from both methods (CT scan and facial image) are utilized, and the transformation matrix is calculated using the 'Create Transformation Matrix' method. The method estimates the scaling, translation and rotation angles from different directions to ensure the identified landmarks from both sources superimpose each other. At 808, the 3D wearable device, the transformation matrix is applied to the 3D holder with all features. This transformation scales and aligns the 3D holder to fit the nose size of the subject. This results in the required 3D wearable device, accurately scaled and ready for use by the subject.

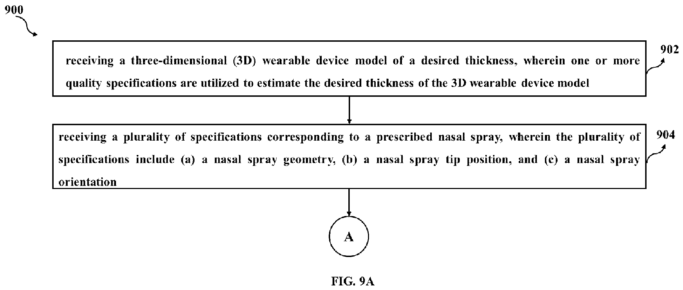

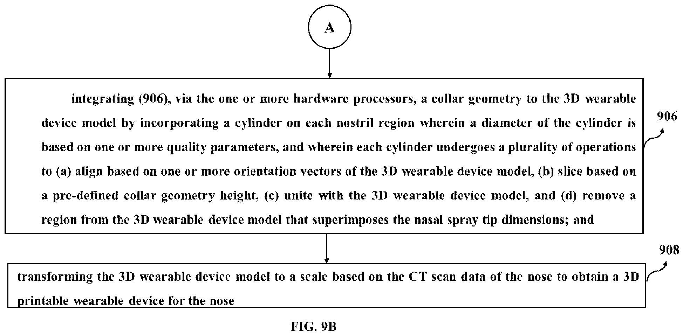

FIGS. 9A-9B is a flow diagram of an illustrative method 900 modeling the 3D wearable device using the system of FIG. 1, according to some embodiments of the present disclosure.

The steps of method 900 of the present disclosure will now be explained with reference to the components or blocks of the system 100 as depicted in FIG. 1 through FIG. 10. Although process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any order practical. Further, some steps may be performed simultaneously. In the present disclosure, modelling of the 3D wearable device for facilitating administration of drugs through nose is provided. The modeling facilitate an introduction of a collared geometry that provides customed passage to the drug within the nasal cavity based on a plurality of nasal spray specifications. Further, the 3D wearable device model is transformed to a scale based on the CT scan data of the nose to obtain a 3D printable wearable device for the nose.

At step 902 of the method 900, the one or more hardware processors 104 are configured to receive a three dimensional (3D) wearable device model of a desired thickness, wherein one or more quality specifications are utilized to estimate the desired thickness of the 3D wearable device model. The 3D wearable device model of the desired thickness is created by receiving (a) one or more two dimensional (2D) facial images of a subject, and (b) a CT scan data of nose of the subject. The plurality of 2D facial images of the subject include at least one front view image, at least one left-side view image, and at least one right-side view image. A plurality of feature points corresponding to the nose of the subject are extracted from the one or more 2D facial images of the subject. Using the extracted plurality of feature point, a zero-thickness nose boundary surface file is generated. In the zero-thickness nose boundary surface file, a desired thickness is imparted to obtain a three-dimensional (3D) holder surface. The 3D nasal holder surface serves as a base to perform a plurality of customization operations to generate a 3D wearable nasal device model. The one or more quality specifications are utilized to estimate the desired thickness of the 3D wearable device model. The one or more quality specifications include stiffness of the 3D printed device, material used in the 3D printing of the device, weight of the final device, surface finish of the device and cost of the final 3D printed device.

Further diameter and height of the collar geometry is obtained using the geometrical details of the spray and quality specifications such as material property and strength requirements of the collar. According to an embodiment, the desired thickness is estimated from a plurality of simulation techniques comprising the finite element analysis (FEA), a finite difference analysis (FDA), an empirical relations, one or more data-based techniques, and one or more machine learning techniques, and wherein the thickness is imparted by firstly offsetting the surface by a half thickness to obtain a thickened surface, and further creating a more thickened surface by thickening a mesh to achieve the desired thickness.

At step 904 of the method 900, the one or more hardware processors 104 are configured to receive a plurality of specifications corresponding to a prescribed nasal spray, wherein the plurality of specifications include (a) a nasal spray geometry, (b) a nasal spray tip position, and (c) a nasal spray orientation. A typical nasal spray formulation consists of the drug suspended or dissolved in an aqueous medium, which is filled into a bottle with a metered spray pump. Pump actuation by the patient delivers drug-laden droplets into the nasal cavity. The pump is an integral part of the whole assembly and plays a crucial role in atomizing and delivering an accurate drug dose. However, despite the well-defined specifications of the pump, operator biases greatly impact the performance. Therefore, nasal spray specifications such as tip of the spray bottle, orientation of the bottle, shape of the bottle, actuation mechanism become responsible for product performance in terms of achieving effective concentration on the target site. The system 100 receives the plurality of nasal spray specifications to consider the geometry associated with the tip from where the spray is being originated. For nasal sprays to deliver its full potential, insights into nasal spray atomization is needed to achieve target drug delivery to specific parts of the nasal cavity. Various other spray specifications include spray content uniformity, spray pattern and plume geometry uniformity, spray delivery repeatability and pump to pump reproducibility, which represent quality and reliability of the nasal spray device over the entire cycle of actuations from a spray bottle.

At step 906 of the method 900, the one or more hardware processors 104 are configured integrating a collar geometry to the 3D wearable device model by incorporating a cylinder on each nostril region wherein a diameter of the cylinder is based on one or more quality parameters, and wherein each cylinder undergoes a plurality of boolean operations to (a) align based on one or more orientation vectors of the 3D wearable device model, (b) slice based on a pre-defined collar geometry height, (c) unite with the 3D wearable device model, and (d) remove a region from the 3D wearable device model that superimposes the nasal spray tip dimensions. The alignment of the cylinder towards outer surface of the 3D wearable device model is based on alignment vectors including an orientation vector and an axis vector. A transformation matrix is calculated using orientation vector and axis vector of the cylinder. The cylinder thus created is transformed to achieve the required orientation. The slicing involves performing one or more boolean operations on the 3D wearable device model to generate the one or more collars. Two collars of the pre-defined height covering the nostril region are protruded towards outer surface of the 3D wearable device model. The 3D thickened holder surface obtained in the previous step is further inflated and offset using one or more quality parameters. The inflation and offset will result in the height of the collar geometry. The one or more collars assists in a nasal drug ingestion to a target site within the nose. Slicing is a customized approach of making geometric changes to the collar based on nasal spray specifications. The slicing generates an intersection volume of the collared geometry. The system 100 utilizes the nasal spray tip dimensions tip dimension received as one of the nasal spray specifications. After slicing operation, the collar geometry is integrated to the 3D wearable device model by a boolean union operation. The orientation of the nasal spray is achieved by performing transformation using transformation matrix. The transformation matrix is calculated using the nasal spray tip position and orientation and tip position and orientation from the received specifications. Based on the nasal spray tip dimensions, a portion of the 3D wearable model is removed by using the boolean operator that creates an opening for the nasal spray. The boolean subtraction operation is carried out on the 3D wearable device model for the opening along the aligned collar. The opening is customized as per nasal spray tip dimensions and facilitate an assisted delivery of the drug to the target site. The creation of cylinder along each nostril involved CAD features wherein a cylinder geometry is aligned and integrated to the 3D wearable device model based on one or more quality specification as well as one or more nasal spray specifications. The elevated structure from a base of the 3D wearable device. That is aesthetically created to provide a decent space surrounding the nostrils for easing out normal breathing. According to an embodiment of the present disclosure, the 3D nasal spray is aligned in the collar of the 3D wearable device model as per the nasal spray tip position and orientation via a Boolean operation to create an opening for dispensing the prescribed nasal spray.

At step 908 of the method 900, the one or more hardware processors 104 are configured to transform the 3D wearable device model to a scale based on the CT scan data of the nose to obtain a 3D printable wearable device for the nose. The 3D face STL file is generated from the CT scan data by classifying the 3D scan data based on the pre-defined threshold, and by cleaning with desired features wherein the classified data is cleaned to retain the desired features. The 3D face of the subject using CT data is generated by using threshold value corresponding to the air threshold value in the CT data. This threshold value provides 3D profile of the face corresponding to skin of the subject. The thresholding also results into various volumes inside head. These volumes are dropped based on the fact that volume size corresponding to the face has largest size as compared to other volumes in the head generated using thresholding. The resulting geometry is saved in STL format for further processing. The STL file generation is shown in FIG. 8. The stl file is used in identifying one or more face landmarks. The landmark identification is done using techniques such as deep learning-based technique on STL file or alternatively a STL is given a skin color and facial snap is generated and facial landmarks are obtained using image landmark techniques. From the image landmark, a plurality of image landmark points are extracted to represent nasal geometry to be captured for the 3D wearable device. The transformation matrix is calculated to scale the 3D wearable device model. For the calculation of the transformation matrix, key landmarks from both methods (CT scan and facial image) are utilized. The method estimates the scaling and rotation angles from different directions to ensure the identified landmarks from both sources superimpose each other. The transformation scales and aligns the 3D holder to fit the real nose size.

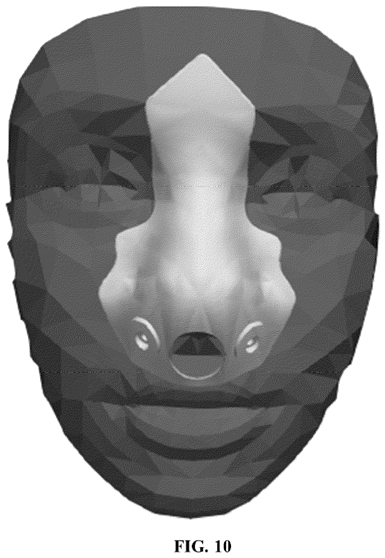

According to an embodiment of the present disclosure, a 3D wearable device for nose of a subject is provided. The 3D wearable device comprises: a body having an inner surface designed in synchronization with one or more external aspects of the nose of the subject, and an outer surface having a plurality of collars to direct a nasal drug delivery to a target site within the nose; and a right-side nostril opening and a left-side nostril opening provisioning the nasal spray entry to the nose, and wherein the 3D wearable device is obtained using one or more 3D printing techniques. An illustration of 3D wearable device customized according to the nasal spray specifications as well as personalized to the subject based on CT scan data is shown in FIG. 10.

According to an embodiment of the present disclosure, the 3D wearable device comprises an earring part configured to detachably fix the 3D wearable device to ears of the subject.

According to an embodiment of the present disclosure, the 3D wearable device model is transformed to the 3D printable wearable device for the nose using one or more 3D printing techniques.

According to an embodiment of the present disclosure, the 3D wearable device is utilized by the subject in one or more ways comprising of: (i) detachably fixing to the ears of the subject, (ii) handheld by the user, and (iii) sticking to nose of the subject at one or more elevated points.

The written description describes the subject matter herein to enable any person skilled in the art to make and use the embodiments. The scope of the subject matter embodiments is defined herein and may include other modifications that occur to those skilled in the art. Such other modifications are intended to be within the scope of the present disclosure if they have similar elements that do not differ from the literal language of the present disclosure or if they include equivalent elements with insubstantial differences from the literal language of the embodiments described herein.

Therefore, modeling of a three-dimensional (3D) wearable device for assisting nasal administration of drugs is provided. The 3D wearable device aid in achieving maximum concentration of the drug at the target site. The modeling facilitate an introduction of a collared geometry that provides customed passage to the drug within the nasal cavity based on a plurality of nasal spray specifications. Further, the 3D wearable device model is transformed to a scale based on the CT scan data of the nose to obtain a 3D printable wearable device for the nose. The 3D wearable device is a personalized or a customizable aid for the intra-nasal drug delivery. The 3D wearable device model is generated by considering nasal anatomy of the subject as well as the nasal spray specifications. The method of generating the 3D wearable device model considers a plurality of features extracted from the facial images of the subject, CT scan data of the subject as well as the specifications of the nasal spray prescribed to the subject. A customized solution in terms of the 3D wearable device model assists the subject in achieving maximum drug concentration at the target site within the nasal cavity.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method (900) for an assisted nasal drug delivery through a personalized device, the method comprising:
receiving (902), via one or more hardware processors, a three dimensional (3D) wearable device model of a desired thickness, wherein one or more quality specifications are utilized to estimate the desired thickness of the 3D wearable device model;
receiving (904), via the one or more hardware processors, a plurality of specifications corresponding to a prescribed nasal spray, wherein the plurality of specifications include (a) a nasal spray geometry, (b) a nasal spray tip dimension, and (c) a nasal spray orientation;
integrating (906), via the one or more hardware processors, a collar geometry to the 3D wearable device model by incorporating a cylinder on each nostril region wherein a diameter of the cylinder is based on one or more quality parameters, and wherein each cylinder undergoes a plurality of operations to (a) align based on one or more orientation vectors of the 3D wearable device model, (b) slice based on a pre-defined collar geometry height, (c) unite with the 3D wearable device model, and (d) remove a region from the 3D wearable device model that superimposes the nasal spray tip dimensions; and
transforming (908), via the one or more hardware processors, the 3D wearable device model to a scale based on the CT scan data of the nose to obtain a 3D printable wearable device for the nose.

2. The method as claimed in claim 1, wherein the 3D wearable device model of the desired thickness is created by a process involving steps:
receiving (a) one or more two dimensional (2D) facial images of a subject;
extracting a plurality of feature points corresponding to the nose of the subject from the one or more 2D facial images of the subject;
generating, via the one or more hardware processors, a zero-thickness nose boundary surface file using the extracted plurality of feature points; and
imparting a desired thickness to the zero-thickness nose boundary surface file to obtain the three dimensional (3D) wearable device model, wherein one or more quality specifications are utilized to estimate the desired thickness of the 3D wearable device model.

3. The method as claimed in claim 1, wherein the one or more 2D facial images of the subject include at least one front view image, at least one left-side view image, and at least one right-side view image.

4. The method as claimed in claim 1, wherein the 3D wearable device model is transformed to the 3D printable wearable device model for the nose using one or more 3D printing techniques, an injection molding technique, a blow molding technique and a machining technique, and wherein the 3D wearable device model is transformed to the 3D printable wearable device model for the nose using the one or more 3D printing techniques comprising a selective laser sintering (SLS), a fused deposition modeling (FDM), a digital light processing (DLP), similar to stereolithography (SLA), a selective laser melting (SLM), a binder jetting, a polyjet, and a laminated object manufacturing.

5. The method as claimed in claim 1, wherein the desired thickness is estimated from a plurality of simulation techniques comprising a finite element analysis (FEA), a finite difference (FDA) analysis, an empirical relations, one or more data-based techniques, and one or more machine learning techniques, and wherein one of the plurality of simulation techniques is imparted by firstly offsetting the surface by a half thickness to obtain a thickened surface, and further creating a more thickened surface by thickening a mesh to achieve the desired thickness.

6. The method as claimed in claim 1, wherein the scaling operation involves:
processing of the CT scan data to generate a first facial landmark comprising a plurality of 3D facial datapoints of the entire face;
processing of the at least one 2D facial image of the face to generate a second facial landmark comprising a plurality of 2D facial datapoints of the entire face;
generating a transformation matrix by combining the first facial landmark and the second facial landmark; and
scaling the transformation matrix to obtain the 3D wearable device model corresponding to the actual size of the nose.

7. A system (100), comprising:
a memory (102) storing instructions;
one or more communication interfaces (106); and
one or more hardware processors (104) coupled to the memory (102) via the one or more communication interfaces (106), wherein the one or more hardware processors (104) are configured by the instructions to:
receive a three dimensional (3D) wearable device model of a desired thickness, wherein one or more quality specifications are utilized to estimate the desired thickness of the 3D wearable device model;
receive a plurality of specifications corresponding to a prescribed nasal spray, wherein the plurality of specifications include (a) a nasal spray geometry, (b) a nasal spray tip dimension, and (c) a nasal spray orientation;
integrate a collar geometry to the 3D wearable device model by incorporating a cylinder on each nostril region wherein a diameter of the cylinder is based on one or more quality parameters, and wherein each cylinder undergoes a plurality of operations to (a) align based on one or more orientation vectors of the 3D wearable device model, (b) slice based on a pre-defined collar geometry height, (c) unite with the 3D wearable device model, and (d) remove a region from the 3D wearable device model that superimposes the nasal spray tip dimensions; and
transform the 3D wearable device model to a scale based on the CT scan data of the nose to obtain a 3D printable wearable device for the nose.

8. The system as claimed in claim 7, wherein the 3D wearable device model of the desired thickness is created by a process involving steps:
receiving (a) one or more two dimensional (2D) facial images of a subject;
extracting a plurality of feature points corresponding to the nose of the subject from the one or more 2D facial images of the subject;
generating, via the one or more hardware processors, a zero-thickness nose boundary surface file using the extracted plurality of feature points; and
imparting a desired thickness to the zero-thickness nose boundary surface file to obtain the three dimensional (3D) wearable device model, wherein one or more quality specifications are utilized to estimate the desired thickness of the 3D wearable device model.

9. The system as claimed in claim 7, wherein the one or more 2D facial images of the subject include at least one front view image, at least one left-side view image, and at least one right-side view image.

10. The system as claimed in claim 7, wherein the one or more 2D facial images of the subject include at least one front view image, at least one left-side view image, and at least one right-side view image.

11. The system as claimed in claim 7, wherein the 3D wearable device model is transformed to the 3D printable wearable device model for the nose using one or more 3D printing techniques, an injection molding technique, a blow molding technique and a machining technique, and wherein the 3D wearable device model is transformed to the 3D printable wearable device model for the nose using the one or more 3D printing techniques comprising a selective laser sintering (SLS), a fused deposition modeling (FDM), a digital light processing (DLP), similar to stereolithography (SLA), a selective laser melting (SLM), a binder jetting, a polyjet, and a laminated object manufacturing.

12. The system as claimed in claim 7, wherein the desired thickness is estimated from a plurality of simulation techniques comprising a finite element analysis (FEA), a finite difference (FDA) analysis, an empirical relations, one or more data-based techniques, and one or more machine learning techniques, and wherein one of the plurality of simulation techniques is imparted by firstly offsetting the surface by a half thickness to obtain a thickened surface, and further creating a more thickened surface by thickening a mesh to achieve the desired thickness.

13. The system as claimed in claim 7, wherein the scaling operation involves:
processing of the CT scan data to generate a first facial landmark comprising a plurality of 3D facial datapoints of the entire face;
processing of the at least one 2D facial image of the face to generate a second facial landmark comprising a plurality of 2D facial datapoints of the entire face;
generating a transformation matrix by combining the first facial landmark and the second facial landmark; and
scaling the transformation matrix to obtain the 3D wearable device model corresponding to the actual size of the nose.

14. The system as claimed in claim 7, wherein the 3D printable wearable device for the nose is transformed to a 3D wearable device using the one or more 3D printing techniques, and wherein the 3D wearable device comprises:
a body having an inner surface designed in synchronization with one or more external aspects of the nose of the subject, and an outer surface having a plurality of collars to direct a nasal drug delivery to a target site within the nose; and
a right-side nostril opening and a left-side nostril opening provisioning the nasal spray entry to the nose, and
wherein the 3D printable wearable device for the nose comprises an earring part configured to detachably fix the 3D wearable device to ears of the subject, and wherein the 3D printable wearable device for the nose is utilized by the subject in one or more ways comprising of: (i) detachably fixing to the ears of the subject, (ii) handheld by the user, and (iii) sticking to nose of the subject at one or more elevated points.

15. One or more non-transitory machine-readable information storage mediums comprising one or more instructions which when executed by one or more hardware processors cause:
receiving a three dimensional (3D) wearable device model of a desired thickness, wherein one or more quality specifications are utilized to estimate the desired thickness of the 3D wearable device model;
receiving a plurality of specifications corresponding to a prescribed nasal spray, wherein the plurality of specifications include (a) a nasal spray geometry, (b) a nasal spray tip dimension, and (c) a nasal spray orientation;
integrating a collar geometry to the 3D wearable device model by incorporating a cylinder on each nostril region wherein a diameter of the cylinder is based on one or more quality parameters, and wherein each cylinder undergoes a plurality of operations to (a) align based on one or more orientation vectors of the 3D wearable device model, (b) slice based on a pre-defined collar geometry height, (c) unite with the 3D wearable device model, and (d) remove a region from the 3D wearable device model that superimposes the nasal spray tip dimensions; and
transforming the 3D wearable device model to a scale based on the CT scan data of the nose to obtain a 3D printable wearable device for the nose.
